# EUROPEAN PATENT APPLICATION

(11) **EP 2 305 100 A1**
(43) Date of publication of application: **06.04.2011**
(21) Application number: 09757656.5
(22) Date of filing: 05.06.2009
(51) Int. Cl.: A61B 3/16

(54) **SENSOR CONTACT LENS, SYSTEM FOR THE NON-INVASIVE MONITORING OF INTRAOCULAR PRESSURE AND METHOD FOR MEASURING SAME**

(30) Priority: 06.06.2008 ES 200801722
(71) Applicant: Consejo Superior De Investigaciones Científicas, 28006 Madrid (ES); Universidad De Valladolid, 47002 Valladolid (ES); Institució Catalana De Recerca I Estudis, 08010 Barcelona (ES); Universitat Autònoma De Barcelona, 08193 Cardanyola del Valles, Barcelona (ES); Ciber-BBN, 50018 Zaragoza (ES)
(72) Inventor: VECIANA, Jaume, E-08193 Bellaterra (Barcelona) (ES); ROVIRA, Concepció, E-08193 Bellaterra (Barcelona) (ES); MAS-TORRENT, Marta, E-08193 Bellaterra (Barcelona) (ES); VILLA SANZ, Rosa, E-08193 Bellaterra (Barcelona) (ES); AGUILÓ LLOBET, Jordi, E-08193 Cerdanyola del Valles (Barcelona) (ES); PASTOR, José Carlos, E-47002 Valladolid (ES); USSA, Fernando, E-47002 Valladolid (ES); LAUKHINA, Elena, E-50018 Zaragoza (ES); LAUKHIN, Vladimir, E-08010 Barcelona (ES); MARTIN HERRANZ, Raul, E-47002 Valladolid (ES); GUIMERA, Anton, E-28006 Madrid (ES)
(74) Representative: Pons Ariño, Angel
(86) International application number: PCT/ES2009/070205
(87) International publication number: WO 2009/147277

(57) **Abstract**

The invention is **characterised in that** it includes a truncated contact lens (1) having a truncation plane parallel to the base of the contact lens and a centrally disposed polymer nanocomposite material (2) joined to the perimeter of the truncated zone, said material being sensitive to pressure changes, biocompatible and transparent and including contact electrodes (3). The invention is also **characterised in that** it includes means for transmitting intraocular pressure measurement data to an external system. The invention also relates to an intraocular pressure measurement method using said lens, consisting of: i) positioning the sensor contact lens on an eye in order to determine the intraocular pressure thereof; ii) supplying a direct current between the external electrodes; iii) measuring ?V between the internal electrodes; and iv) identifying if the value obtained is outside the linear response expressed as changes in resistivity of the polymer nanocomposite material. The invention further relates to a telemetry system including said lens.

## Description

### FIELD OF THE INVENTION

The present invention relates to a sensor contact lens for the monitoring of intraocular pressure (IOP) using a non-invasive technique and a method for measuring intraocular pressure using such sensor contact lens.

In particular, the invention relates to a sensor contact lens comprising in the central area a transparent polymeric nanocomposite material which continuously and noninvasively takes direct measurements of intraocular pressure.

The invention also relates to a telemetry system for monitoring intraocular pressure comprising said sensor contact lens.

### BACKGROUND OF THE INVENTION

Glaucoma is an eye condition due to an increase in intraocular pressure (IOP). This increase results in a slow irreversible damage to the optic nerve which is very difficult to detect in early stages and is also difficult to control because of the numerous fluctuations in IOP throughout the day. Therefore, glaucoma is the second leading cause of visual impairment or blindness in the industrial sector.

The diagnosis and control is performed by measuring the IOP and the most common measurement method used is the Goldmann applanation tonometry.

Thus, patents U.S. 6,994,672 and U.S. 7,169,106, both of the same owner and inventor, disclose devices for measuring intraocular pressure of the eye based on the above technique. They involve a specific measurements or measurements with a certain amount of time between them and, therefore, noncontinuous. In the specific case of U.S. Patent 7,169,106 it discloses a contact lens with a sensor adhered to a part of its inner surface. Said sensor comprises a surface in contact with a portion of the eye surface. The contact surface includes an outer region and an inner region manufactured as an impedance element so that the impedance varies as the inner region changes shape due to the pressure from an outer applanator on its surface modifying said shape and, therefore, varying the impedance.

Furthermore, patent application WO/2003/001991 has developed sensor lenses based on micro-fabricated strain gauge on a polyimide substrate inserted in the outer area of a silicone lens. The IOP measuring system comprises a contact lens, made of silicone for instance, and an active strain gauge attached to said contact lens and is **characterized in that** the active tension gauge is a circular arc and is located on the outside area and around the C centre of the contact lens.

Said contact lens correlates the spherical distortion of the eye to IOP changes and, therefore, IOP is measured indirectly and in terms of curvature changes of the cornea of the human eye of approximately 3µm, within a typical radius of 7.8 mm. In addition, this measurement system and its accuracy depend on the eye movements, blinking and lens movements. This implies signal filtering to remove noise, and factors such as corneal thickness and rigidity or astigmatism, among others, which also affect the measurement accuracy and are more difficult to control.

It is noteworthy that the material used in said international patent application is polyimide-silicone, a hydrophobic material that causes problems with the liquid surface of the eye.

Thus, the systems disclosed to date measure only the changes in IOP and do not involve a direct measurement sensor that could be considered an absolute pressure sensor.

Therefore, the prior art has not yet disclosed a device such as a sensor contact lens non-invasively taking direct and continuous IOP measurements which also overcomes the drawbacks of the techniques disclosed to date.

### BRIEF DESCRIPTION OF THE INVENTION

In a first aspect the present invention relates to a non-invasive sensor contact lens, for directly and continuously monitoring intraocular pressure (IOP) comprising a polymeric nanocomposite material being sensitive to changes in pressure, biocompatible and transparent.

In a second aspect, the invention relates to a method for measuring intraocular pressure (IOP) using the sensor contact lens according to the first aspect of the invention.

The invention also relates in a third aspect to a telemetry system for monitoring intraocular pressure comprising said sensor contact lens.

### FIGURES

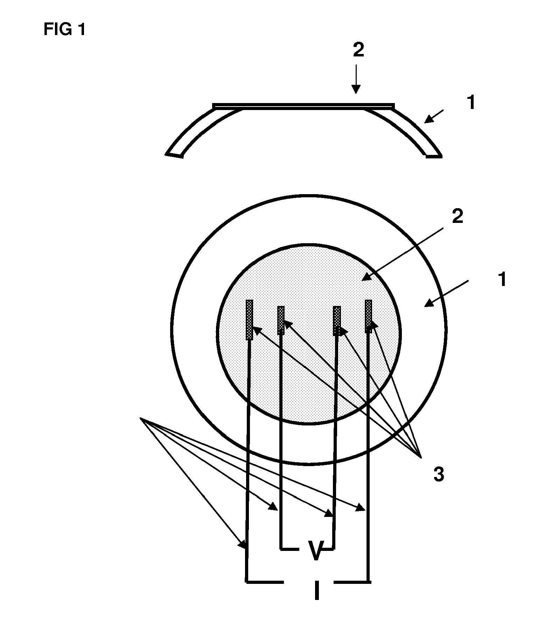

Figure 1 shows a sensor contact lens according to the invention for continuously and non-invasively monitoring the intraocular pressure comprising a truncated contact lens 1, whose truncation plane is parallel to the base of such contact lens, and a centrally disposed polymeric nanocomposite material (2) attached to the perimeter of the truncated area. Said material includes contact electrodes 3 and said truncated contact lens 1, means for transmitting 4 IOP measurement data to an external system (not shown). In said embodiment the means of transmission are wires.

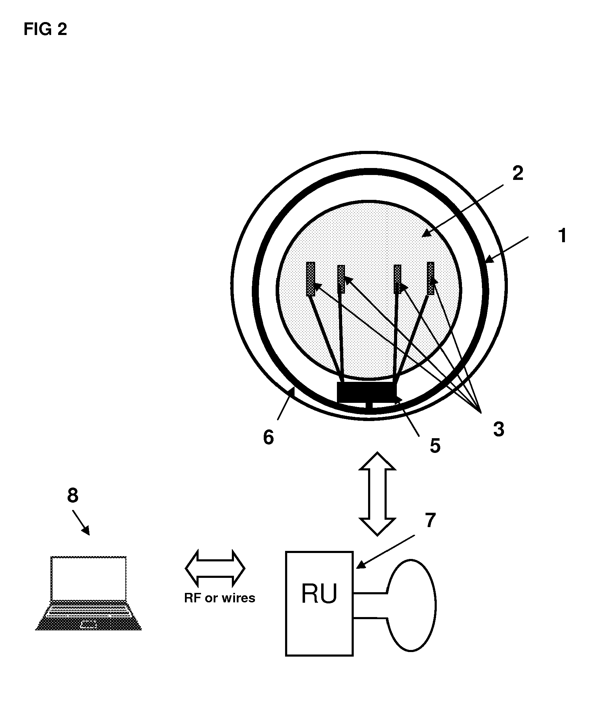

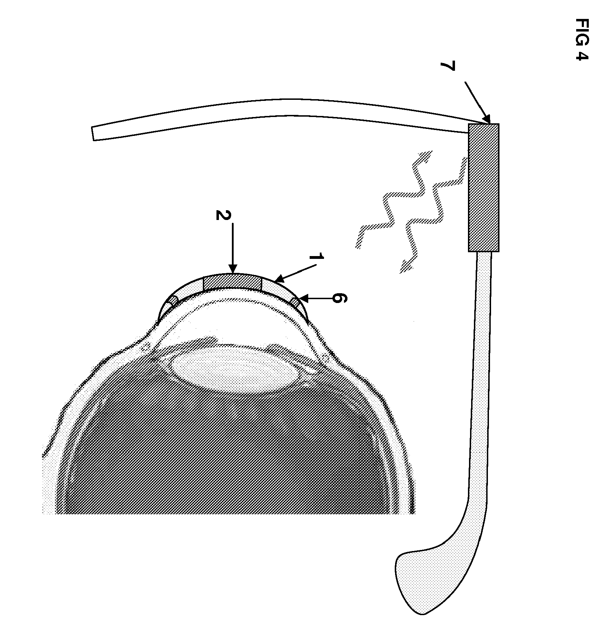

Figure 2 shows an embodiment of the telemetry system of the invention wherein the means of transmission by telemetry include an integrated circuit 5 and an antenna 6. Said figure 2 schematically shows the configuration of a sensor contact lens according to the first aspect of the invention wherein the organic nanocomposite material 2 is connected to an integrated circuit 5 which, via an antenna 6, sends the data to a receiving unit (RU) 7. This unit can be located on a support such as, for example, glasses as shown in Figure 4 below. In addition this unit (RU) provides power to the integrated circuit and via radio frequency (RF) or wires can send the information stored in a PC or PDA (personal device assistant) type data processing unit 8. This unit allows the handling, storage and visualization of data.

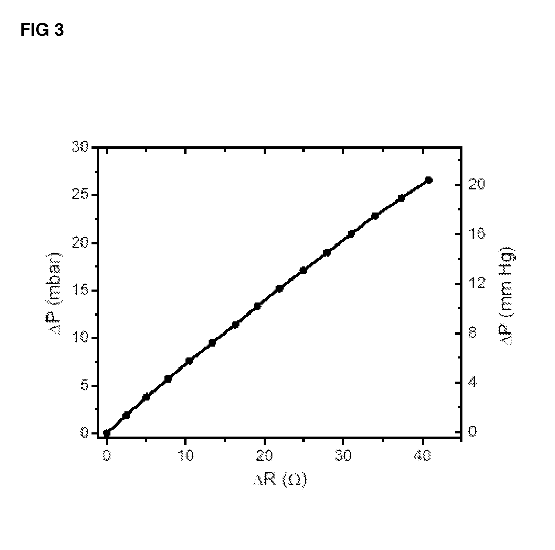

Figure 3 shows the response of an embodiment of the ocular sensor lens according to the first aspect of the invention during a variation in the intraocular pressure (IOP) in terms of resistivity changes with a polymeric nanocomposite material of (BET-TTF)₂ IxBr₃-x on a polycarbonate base support.
Figure 4 shows an embodiment of the sensor contact lens of the invention placed on the eyeball and the operation diagram of the system with telemetry elements incorporated in an eyeglass frame, where the references have the meanings given above.

### DETAILED DESCRIPTION OF THE INVENTION

The first aspect of the invention provides a sensor contact lens, of polymethylmethacrylate for instance, to monitor intraocular pressure (IOP), **characterized in that** it comprises a truncated contact lens (1), whose truncation plane is parallel to the base of said contact lens, and a polymeric nanocomposite material (2) centrally disposed and attached to the perimeter of the truncated area, said material being sensitive to pressure changes, biocompatible and transparent, and including contact electrodes (3), and in that it also comprises means for transmitting IOP measurement data to an external system.

The means for transmitting IOP measurement data comprise either wires 4 or an integrated circuit 5 and an antenna 6, wherein said antenna 6 can be located in the truncated lens 1 or as a bonding element with the polymer nanocomposite material 2. Said antenna can be made of platinum, gold or a polymeric nanocomposite material.

On the other hand, the contact electrodes 3 comprise two external electrodes to supply continuous power to the polymeric nanocomposite material and two internal electrodes to measure the differential voltage thereof.

The polymeric nanocomposite material 2 has been obtained from a polymer matrix (base substrate) covered with a layer of organic conductive material intimately linked to the polymer matrix. The conductive layer is formed by a mesh/grid of crystals of a molecular conductor based on a charge-transfer salt.

Advantageously, the use of said polymeric nanocomposite material enables a linear response between changes in intraocular pressure (IOP) in terms of resistivity changes.

Said polymeric nanocomposite material is sensitive to changes in pressure, is biocompatible and transparent and, in particular, is obtained from:
i) a layer of conductive organic material consisting of at least one salt or conductive complex comprising a molecule A and a dopant D, said molecule A being an organic molecule or macromolecule, being an electron donor or acceptor capable of forming a salt or a conductive complex, but which without being doped has no conductivity, and said dopant D being an electron acceptor or donor compound capable of forming a salt or conductive complex with the molecule or macromolecule A; and
ii) a base substrate or polymer matrix, in intimate contact with the layer of organic material i), wherein said base substrate is inert to the layer of organic material i).

Such a molecule or macromolecule A will be selected from between a derivative of acene, of coronene, of tetrathiafulvalene or of tetracyanoquinodimethane, preferably bis (ethylenthio) tetrathiafulvalene (BET-TTF) or bis (ethylenedithio) tetrathiafulvalene (BEDT-TTF). Said dopant D is a halogen species, advantageously being a species selected from iodine, bromine or iodine bromide.

Preferably, said salt is selected from between (BET-TTF)₂I₃, (BET-TTF)₂Br·3H₂O, (BET-TTF)₂IxBr₃-x, and (BET-TTF)₂IxBr₃-x, where BET-TTF is bis (ethylenthio) tetrathiafulvalene and BEDT-TTF is bis (ethylenedithio) tetrathiafulvalene, preferably being (BET-TTF)₂Br·3H₂O, and said base substrate which is inert to the conductive layer of organic material is selected from a non-conductive organic polymer, preferably a thermoplastic polymer or elastomer, more preferably, polycarbonate, polyamide, polymethylmethacrylate, polyethylene or polypropylene.

For pressure sensor applications the substrate having a high resistance to loads mechanically applied in cycles and that difficult to break through load application will be preferred. Also for this application it will be preferable to have an organic layer sensitive to changes in pressure, distortion or stress consisting of a high-piezoresistive material with a low heat resistance coefficient, preferably being (BET-TTF)₂Br·3H₂O as an organic layer.

For a better understanding of the obtaining of polymeric nanocomposite material 2, the content of Spanish patent application P200602887 is included.

Advantageously, the sensor contact lens according to the first aspect of the invention can take non-invasive direct pressure values over extended periods of time. In addition, the polymeric nanocomposite material 2 used avoids the disadvantages of prior art relating to the cornea thickness.

Also advantageously, said polymeric nanocomposite material 2 not only acts as a pressure sensor but its composition allows the integrated telemetry circuitry to be designed on its surface in order to extract the signals and thus provide a sensor contact lens that is easy to use and allow IOP monitoring in the most physiological manner possible.

So, by means of a thermo printing technique electronic circuits can be designed directly on the surface of polymeric nanocomposite material 2. For a better understanding of the thermo-chemical printing technique the contents of the international patent application W02007/014975 is incorporated by way of reference.

It is noteworthy that according to the sensor contact lens according to the invention, it provides a direct measurement system of intraocular pressure of the human or animal eye overcoming the drawbacks of the techniques disclosed so far.

In particular, according to the first aspect of the invention, the intraocular pressure is transferred directly to the deformation of the entire polymeric nanocomposite material thus changing its resistance and, therefore, directly measuring the intraocular pressure changes. The polymeric nanocomposite material 2 is much more sensitive to changes in pressure and therefore more deformable than the truncated contact lens 1 which surrounds it due to its Young's modulus. In addition, said polymeric nanocomposite material is totally organic and therefore, better for disposal.

The sensor contact lens according to the first aspect of the invention shows a linear response and high sensitivity to pressure in the pressure range required for measuring the intraocular pressure of the eye between 10 and 21 mmHg.

In a second aspect, the invention relates to a method for measuring intraocular pressure (IOP) using the sensor contact lens according to the first aspect of the invention. Said method comprises the following steps:
i) placing said sensor contact lens on an eye to determine its intraocular pressure;
ii) providing a direct current value between the external contact electrodes;
iii) measuring the differential voltage ΔV between the internal contact electrodes ;
iv) identifying whether the value obtained is outside the linear response, expressed in resistivity changes, between the resistance dependence and the pressure of said polymeric nanocomposite material.

In a preferred embodiment of the invention, the polymer nanocomposite material 2 of said lens is a molecular conductor of (BET)₂IxBr₃-x on a polycarbonate base substrate that gives the linear response defined in Figure 3 below.

Direct current values between the external contact electrodes are typically between 10 and 100 µA.

The identification of whether the value obtained in step iv) is beyond the linear response of the polymeric nanocomposite materials defined in accordance with the present invention is carried out by telemetry transmission to a receiver unit (RU) 7 that is sent to a PC or PDA (*personal device assistant*) 8 via radiofrequency (RF) or wires.

According to the second aspect of the invention, step iv) of the method of measuring intraocular pressure is an essential step to determine the existence of the disease known as glaucoma, such step taking place outside the human or animal body.

The invention also relates in a third aspect to a telemetry system comprising said sensor contact lens. This system is **characterized in that** it comprises a sensor contact lens according to any of claims 1 to 8, a receiving unit (7) for receiving data which, via radio frequency (RF) or wires, sends information to a PC or PDA type data processing unit (8) for the handling, storage and visualization of data.

### EMBODIMENT OF THE INVENTION

The following example embodiment describes the system for the non-invasive monitoring of intraocular pressure (IOP) object of the invention and methodology of use in humans or animals.

The aim of the implementation of the system for the non-invasive IOP monitoring object of the invention is to allow continuous monitoring, for 24 hours for example, to assess the pressure changes that occur throughout the day and can be very marked depending on the hour (circadian rate) or due to medication effects. These fluctuations are difficult or impossible to detect with specific measurements. This sensor monitoring object of the invention is very accurate and physiologically distinct from other systems previously known. Humans targeted by this system are mainly people susceptible to glaucoma where the fluctuations can be very important.

### DESCRIPTION OF THE MEASURING SYSTEM OF THE PIO

This example uses the IOP monitoring system object of the invention comprising the following configuration:

A sensor contact lens for non-invasively measuring IOP, consisting of the sensor contact lens model with wires according to Figure 1. A standard ocular lens cut parallel to the base of the lens, leaving a perimeter of 6 mm in diameter where the transparent organic polymer nanocomposite material is attached and presenting four gold electrodes of 0.3 mm in diameter and 1 mm apart. Said electrodes are connected to the measuring system through wires.

A four-wire device for measuring the strength of the sensor material has been used (e.g., Agilent 34970A multimeter source meters, Keithley 2400 source meters, Keithley 2601 source meters). Current (DC) is injected, between 10 and 100µA,- between the two external electrodes (I + and I-) and the voltage difference between the internal electrodes (V + and V-) is measured.

The values obtained show resistance variations (Ω) with respect to changes in pressure (mmHg). These values will relate to the pressure values according to a resistance-pressure correlation table shown in Figure 3 for a polymer nanocomposite material in a molecular conductor of (BET-TTF)₂IxBr₃-x on a polycarbonate base substrate.

### DESCRIPTION OF THE METHODOLOGY

### Calibration:

Prior to its placement in the eye the IOP will be measured using standard equipment: Goldmann applanation tonometer, and will be taken as the reference value since the system monitors the relative pressure values.

### Handling and measurements:

The sensor contact lens will then be placed as if it were a normal ocular lens. This example uses the wire model and therefore care must be taken to route the wires to the outer edge of the eye so the lid can open and close without any interference.

The wire will be connected from the polymeric nanocomposite material 2 to the meter. Data will be recorded for 24 hours. The data are relayed to a PC for storage, filtration and analysis.

### Values calculation:

Finally, data is displayed on a 24-hour graph calibrated with the initial absolute value recorded.

Normal values of IOP fluctuate throughout the day due to the circadian rhythm. IOP values are between 10 and 21 mmHg. Peaks or increased elevations beyond baseline values will involve therapeutic changes for the patient to avoid injury to the optic nerve.

During monitoring, patient's vision remains normal because the sensor lens is transparent.

In the model which incorporates the telemetry (see Figure 2) a human can continue with his or her everyday life and the IOP measure will reflect even better the normal physiological conditions the of person whose IOP is measured.

## Claims

1. sensor contact lens for the non-invasive monitoring of intraocular pressure (IOP), **characterized in that** it comprises a truncated contact lens (1), which truncation plane is parallel to the base of said contact lens, and a polymeric nanocomposite material (2) centrally arranged and attached to the perimeter of the truncated area, said material being responsive to pressure changes, biocompatible and transparent, and including contact electrodes (3), and **in that** it also comprises means of transmitting IOP measurement data to an external system.

2. Sensor contact lens according to claim 1, wherein said polymeric nanocomposite material is obtained from:
i) a layer of organic material made up of at least one salt or conductive complex comprising a molecule A and a dopant D, said molecule A being to an organic molecule or macromolecule, being an electron donor or acceptor capable of forming a salt or conductive complex, but having no conductivity without being doped, and said dopant D being an electron acceptor or donor compound capable of forming a salt or conductive complex with the molecule or macromolecule A; and
ii) a base substrate or polymer matrix, in intimate contact with said layer of organic material i), wherein said base substrate is inert to said layer of organic material i).

3. Sensor contact lens according to claim 1 or 2, wherein said polymeric nanocomposite material (2) is based on (BET-TTF)2IxBr3-x on a polycarbonate polymeric matrix.

4. Sensor contact lens according to claim 1, wherein said truncated contact lens (1) is made of polymethylmethacrylate.

5. Sensor contact lens according to claim 1, wherein said means of transmitting IOP measurement data comprise either wires (4) or an integrated circuit (5) and an antenna (6).

6. Sensor contact lens according to claims 1 and 5, wherein said antenna (6) is located in the truncated contact lens (1).

7. Sensor contact lens according to claim 6, wherein said antenna (6) is made of platinum, gold or said polymeric nanocomposite material.

8. Sensor contact lens according to claim 1, wherein said contact electrodes (3) are two external electrodes for supplying direct current and two internal electrodes for measuring the differential voltage.

9. Method for measuring intraocular pressure (IOP) using a sensor contact lens according to any of claims 1 to 8 comprising the following steps:
i) placing said sensor contact lens in the eye to determine its intraocular pressure;
ii) supplying a direct current value between the external contact electrodes;
iii) measuring the differential voltage ΔV between the internal contact electrodes;
iv) identifying whether the value obtained is outside the linear response, expressed in changes of resistivity, between the dependence of the resistance and the pressure of said polymeric nanocomposite material.

10. Method for measuring intraocular pressure (IOP) according to claim 9, wherein, said polymeric nanocomposite material being based on a molecular conductor of (BET-TTF)₂IxBr₃-x on a polycarbonate base substrate, the linear response defined in Figure 3 is obtained.

11. System for monitoring intraocular pressure, **characterized in that** it comprises a sensor contact lens according to any of claims 1 to 8, a data receiving unit (7) which sends information to a PC or PDA (8) type data processing unit via radio frequency (RF) or wires for data handling, storage and visualization.
